# EUROPEAN PATENT APPLICATION

(11) **EP 2 107 377 A1**
(43) Date of publication of application: **07.10.2009**
(21) Application number: 08154109.6
(22) Date of filing: 04.04.2008
(51) Int. Cl.: G01N 33/74, G01N 33/68

(54) **Pro-endothelin-1 levels for the prediction of risk of tachyarrhytmic events**

(71) Applicant: BRAHMS Aktiengesellschaft, 16761 Hennigsdorf (DE)
(72) Inventor: Struck, Joachim, 13465 Berlin (DE); Morgenthaler, Nils, 13503 Berlin (DE); Bergmann, Andreas, 12351 Berlin (DE); Müller, Christian, 4031 Basel (CH)
(74) Representative: Kilger, Ute

(57) **Abstract**

The invention relates to *in vitro* methods for prognosis and risk-stratification for a patient having a cardiac disease as heart failure. The invention further relates to the use of such a method.

## Description

The invention relates to *in vitro* methods for risk stratification for indication of device implantation, and/or antiarrhythmic hybrid therapy for a patient having a cardiac disease such as heart failure. The invention further relates to the use of such a method.

Implantable cardioverter defibrillators (ICD) effectively terminate malignant ventricular arrhythmias averting sudden death. However, ICD implantation is expensive, invasive, and associated with adverse effects such as inappropriate shocks, psychosocial problems, lead problems, and infection (Ezekowitz et al, Ann Intern Med 2007;147:251-62; Rosenqvist et al, Circulation 1998;98:663-70; Sanders GD et al, N Engl J Med 2005;353:1471-80). Of interest, the cost-effectiveness of prophylactic implantation of an ICD has even been questioned (Goldman et al, N Engl J Med 2005;353:1513-5), since up to two thirds of patients die without receiving prior ICD therapy (Ezekowitz et al, Ann Intern Med 2007;147:251-62). Consistently, risk stratification for proper indication of device implantation, and/or antiarrhythmic hybrid therapy is mandatory (Ezekowitz et al, Ann Intern Med 2007;147:251-62).
Activation of the neurohumoral system has been linked to increased risk for malignant tachyarrhythmias (fast ventricular tachycardia or ventricular fibrillation) in patients with systolic left ventricular (LV) dysfunction. Indeed, increased levels of B-type natriuretic peptide (BNP) indicate increased risk of sudden cardiac death (Berger et al, Circulation 2002;105:2392-7; Brunner-La Rocca et al, Eur Heart J 2001;22:1136-43). However, BNP is a quantitative marker of the severity of heart failure and indicates increased risk of death (Dao et al, J Am Coll Cardiol 2001;37:379-85), while it is not useful to correctly predict tachyarrhythmic events requiring ICD therapy before.

Thus, it was an object of the present invention to find a marker predicting tachyarrhythmic events in patients having a cardiac disease as e.g. chronic heart failure.

Surpisingly, it was found that increased levels of CT-proET-1 may predict the occurrence of a first malignant ventricular tachyarrhythmic event requiring device therapy. Increased levels of CT-proET-1 independently predict the future occurrence of malignant tachyarrhythmias in ICD patients with heart failure and reduced LV-EF. Further, measurement of CT-proET-1 levels may be very useful for risk stratification of patients requiring an ICD for primary prevention of sudden arrhythmic death.

Thus, subject of the present invention is an *in vitro* method for risk stratification for indication of ICD device implantation, and/or antiarrhythmic hybrid therapy for a patient having a cardiac disease comprising:
- determining the level of Pro-Endothelin-1 (ProET-1) or fragments thereof of at least 12 amino acids in a sample obtained from a patient.

In case the level of fragments of ProET-1 are determined these fragments comprise at least 12 amino acids, preferably at least 20 amino acids, were preferably at least 30 amino acids. Endothelin (ET)-1 is a potent endothelium-derived endogenous vasoconstrictor (Yanagisawa M, Kurihara H, Kimura S, Goto K, Masaki T. A novel peptide vasoconstrictor, endothelin, is produced by vascular endothelium and modulates smooth muscle Ca2+ channels. J Hypertens Suppl 1988;6:S188-91.). ET-1 exerts its vascular effects by activation of ET(A) and ET(B) receptors on smooth muscle cells, which causes an increase in intracellular calcium (Yanagisawa et al, J Hypertens Suppl 1988;6:S188-91). Mature Endothelin-1 is derived from a larger precursor termed Pro-Endothelin-1. Pro-Endothelin-1 can be proteolytically processed into various fragments as described (Struck J, Morgenthaler NG, Bergmann A. Proteolytic processing pattern of the endothelin-1 precursor in vivo. Peptides. 2005 Dec;26(12):2482-6.). These fragments are subject to proteolytic degradation in the blood circulation, which can happen quickly or slowly, depending on the type of fragment and the type and concentration/activity of proteases present in the circulation. One example of these fragments is C-terminal pro-Endothelin-1 (CT-proET-1), which can be measured by a sandwich immunoassay (Papassotiriou J, Morgenthaler NG, Struck J, Alonso C, Bergmann A. Immunoluminometric assay for measurement of the C-terminal endothelin-1 precursor fragment in human plasma. Clin Chem. 2006 Jun;52(6):1144-51.)

Continuous variables are expressed as mean±standard deviation (SD) or expressed as median (range) and point estimate (95% confidence intervals) as indicated. The objective was to examine whether CT-proET-1 contributes to predict time to first malignant tachyarrhythmic event. Malignant tachyarrhythmias were defined as fast ventricular tachyarrhythmias (VT or VF) with cycle lengths ≤ 250ms (heart rate ≥240 bpm) probably leading to death if not terminated by the device (Bocker D, Block M, Isbruch F, et al. Do patients with an implantable defibrillator live longer? J Am Coll Cardiol 1993;21:1638-44.). Univariate and multivariable Cox proportional hazards models were used to evaluate the associations between the outcome measures.

In one embodiment of the method according to the present invention said level of Pro-Endothelin-1 (ProET-1) or fragments thereof of at least 12 amino acids is correlated to the risk of first malignant tachyarrhythmias.
The objective of said method is the stratification of patients according to their requirement for an implantable cardioverter defibrillator (ICD), and/or an antiarrythmic hybrid therapy.

According to a preferred embodiment of the invention said correlating step comprises comparing said level of Pro-Endothelin-1 (ProET-1) or fragments thereof of at least 12 amino acids to a threshold level, whereby, when said level of Pro-Endothelin-1 (ProET-1) or fragments thereof of at least 12 amino acids exceeds said threshold level, said patient is at high risk of tachyarrhythmia.

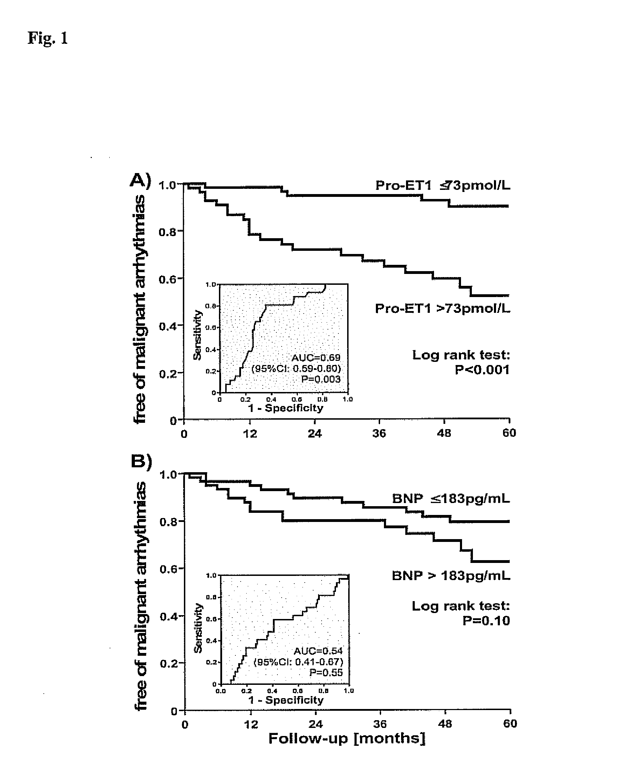

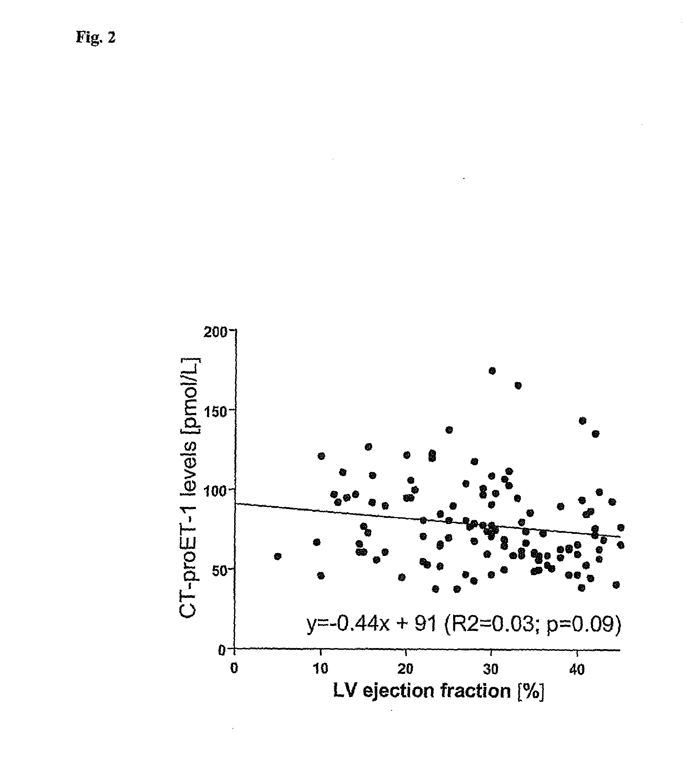

ICD patients have been stratified into two groups according to CT-proET-1 or BNP levels and Kaplan Meier analysis has been performed. Risk of tachyarrhythmia was significantly higher in patients with CT-proET-1 plasma levels >73pmol/L (median) than in patients with CT-proET-1 values below this cut point (logrank test, p<0.001, Fig. 1A). No significant differences were found in patients with BNP plasma levels ≤183 pg/mL (median) or >183pg/mL using malignant tachyarrhythmia as endpoint (logrank test, p=0.10, Fig. 1B).. No significant associations of LV ejection fraction and CT-proET-1 levels were found (Fig. 2).
The area under the ROC curve that used CT-proET-1 levels to predict the occurrence of a first malignant tachyarrhythmia was 0.69 (Fig. 1A, insert). The optimum concentration of CT-proET-1 for the calculation of positive and negative predictive accuracy as obtained from the ROC curve was 75.5 pmol/L (sensitivity 80.8%, specificity 64.4%, negative predictive value 92.1 %, positive predictive value 39.6%, and a positive likelihood ration of 2.27).

CT-proET-1 plasma levels significantly contributed to multivariable Cox regression models predicting the risk of first malignant tachyarrhythmia. Risk ratio has been calculated per pmol/L increment of CT-proET-1 levels (Table 2). Using log CT-proET-1 in statistical analysis did not change obtained results. Multivariable Cox regression analysis revealed that treatment with betablockers indicates favourable prognosis (Table 2).
Thus, in a preferred embodiment of the invention said threshold level is at about 73 +/- 20% pmol/L.

According to a preferred embodiment the cardiac disease is any condition, for which the implantation of an ICD is potentially indicated, inlcuding heart failure of either ischemic or non-ischemic etiology. According to a preferred embodiment the patients have a LV ejection fraction ≤45%.

The sample obtained from a patient is a sample selected from the group comprising a blood sample, a serum sample, and a plasma sample.

Further, a subject of the present invention is a method further comprising combining said level of Pro-Endothelin-1 (ProET-1) or fragments thereof of at least 12 amino acids with the level/condition of one or more additional prognostic markers, parameters or factors, whereby the combination of said level of Pro-Endothelin-1 (ProET-1) or fragments thereof of at least 12 amino acids with said level/condition of one or more additional prognostic markers, parameters or factors increases the predictive value for risk of said method.

In a preferred embodiment, the additional prognostic marker, parameter or factor is selected from a group comprising all parameters listed in Table 2, which exhibit a p<0.05 in the univariate or multivariate analysis, whereby BNP is only an example for proBNP or fragments thereof of at least 12 amino acids including BNP or NT-proBNP.

Preferably, said level of Pro-Endothelin-1 (ProET-1) or fragments thereof of at least 12 amino acids is measured with a sandwich immunoassay. Preferably, C-terminal ET-1 precursor fragment (CT-proET-1), which indirectly estimates activity of the endothelin system, is measured. In a preferred embodiment 2 antibodies are used in this assay, preferably polyclonal antibodies. In a preferred embodiment the antibodies are directed to amino acids 168-212 of pre-proET-1. This method may be conducted with a commercially available sandwich immunoassay (CT-proET-1 LIA, B.R.A.H.M.S AG, Hennigsdorf, Berlin, The assay (normal reference range: median: 44.3, range: 10.5-77.4 pmol/L) has an analytical detection limit of 0.4 pmol/L and the intra-assay CV were <10% for values >10 pmol/L (Papassotiriou J, Morgenthaler NG, Struck J, Alonso C, Bergmann A. Immunoluminometric assay for measurement of the C-terminal endothelin-1 precursor fragment in human plasma. Clin Chem 2006;52:1144-51.).

### Figure Description

### Figure 1

Heart failure patients have been stratified into two groups according to CT-proET-1 and BNP levels and Kaplan Meier analyses have been performed. A) Patients with CT-proET-1 plasma levels >73pmol/L (median) had a significantly worse outcome than patients with CT-proET-1 values below this cut point (logrank test) regarding malignant tachyarrhythmic events as endpoint. B) Patients with BNP plasma levels ≥ 183 pg/mL (median) did not have a higher rate of malignant ventricular tachyarrhythmic events than patients below this cut point (logrank test). Respective ROC curve analyses are shown in the inserts.

### Figure 2

Linear regression analysis of LV ejection fraction and CT-proET-1 levels in 123 heart failure patients with implantable cardioverter defibrillators.

### Examples

### Methods

### Patients, study design

The study population consisted of 123 defibrillator patients with heart failure of ischemic or nonischemic etiology that were prospectively recruited at the defibrillator outpatient clinic of the University of Marburg between June 2002 and September 2003 (Christ et al, Eur J Heart Fail 2007;9:272-9). The study was conducted according the principles of Good Clinical Practice and the Declaration of Helsinki, and patients gave their informed consent before enrolment into the study (Christ et al, Eur J Heart Fail 2007;9:272-9).

### Electrocardiography and echocardiography

Standardised 12-lead electrocardiograms (ECGs) were recorded at a paper speed of 50 mm/second from each patient and interpreted by a single experienced researcher blinded to outcome. Patients were classified to have pacemaker rhythm, if more than 50% of beats were paced during ECG recording. Two-dimensional echocardiographic images of the heart were obtained at the end of the expiratory period using a Vingmed Vivid Five machine (GE Medical Systems, Solingen, Germany) and parameters measured using standard procedures (Christ et al, Eur J Heart Fail 2007;9:272-9).

### Laboratory Procedures

After overnight fasting, venous blood samples were drawn between 8:30h and 10:30h through a 21-gauge cannula inserted into an antecubital vein using ethylenediamine tetraacetic acid (EDTA) containing monovettes (Sarstedt, Nuembrecht, Germany). Patients lay for at least 15 min in a supine body position. EDTA blood samples were immediately transferred on chilled ice, plasma separated using a centrifuge and plasma stored at -80 °C until analysis.
BNP levels were determined using a chemiluminescence immunoassay (Bayer AG, Fernwald, Germany). The analytical detection limit of the assay was 2 pg/mL. The intra-assay coefficient of variations (CVs) were 1.8-4.3% at 29.4-1763 pg/mL and the interassay CVs were 2.3-4.7% at 29.4-1736 pg/mL BNP (Christ et al, Eur J Heart Fail 2007;9:272-9). Endothelin-1 is difficult to measure due to instability and receptor binding. C-terminal ET-1 precursor fragment (CT-proET-1), which indirectly estimates activity of the endothelin system, was measured with a novel sandwich immunoassay (CT-proET-1 LIA, B.R.A.H.M.S AG, Hennigsdorf, Berlin, Germany) (Papassotiriou et al, Clin Chem 2006;52:1144-51)) using 2 polyclonal antibodies directed to amino acids 168-212 of pre-proET-1. The assay (normal reference range: median: 44.3, range: 10.5-77.4pmol/L) has an analytical detection limit of 0.4pmol/L and the intra-assay CV were <10% for values >10pmol/L (Papassotiriou et al, Clin Chem 2006;52:1144-51).

### Defibrillator implantation and device programming

Exclusively, ICDs with non-thoracotomy lead systems and biphasic shock waveforms with maximum shock energy of 27 to 34 J were used. All ICD systems used provided stored intracardiac electrograms in addition to beat-to-beat intervals of episodes triggering device action (Guidant, St. Paul, Minnesota; Medtronic, Minneapolis, Minnesota). The time interval for VF detection ranged from one to three seconds in Guidant devices; the Medtronic devices were programmed to require 18 out of 24 beats below the programmed detection cycle length (range 240 - 280ms) in order to initiate capacitor charging in the VF zone. Slower ventricular tachyarrhythmias were detected and treated in one or two separate VT zones with antitachycardia burst pacing followed by up to 4 cardioversion shocks (Grimm et al, J Am Coll Cardiol 2002;39:780-7).

### Follow-up

All data of baseline clinical characteristics, including the results of cardiac evaluation and implantation data had been collected prospectively in the Marburg Defibrillator Database (Christ et al, Eur J Heart Fail 2007;9:272-9, Grimm et al, J Am Coll Cardiol 2002;39:780-7).

Follow-up in this study started at the time of biomarker measurement, was up to 60 months and could be completed in all patients enrolled. Patients were followed primarily in our defibrillator outpatient clinic in three- to six months intervals or as soon as possible after spontaneous ICD shocks for device interrogation and retrieval of stored electrocardiograms. Two experienced electrophysiologists classified all stored electrocardiograms of episodes triggering ICD therapy or inappropriate using previously described criteria (Christ et al, Eur J Heart Fail 2007;9:272-9; Marchlinski et al, Pacing Clin Electrophysiol 1993;16:527-34).

### Statistical Analysis

Continuous variables are expressed as mean±standard deviation (SD) or expressed as median (range) and point estimate (95% confidence intervals) as indicated. The primary objective was to examine whether biomarker levels including CT-proET-1 or BNP contribute to predict time to first malignant tachyarrhythmic event. Malignant tachyarrhythmias were defined as fast ventricular tachyarrhythmias (VT or VF) with cycle lengths ≤250ms (heart rate ≥240 bpm) probably leading to death if not terminated by the device (Bocker et al, J Am Coll Cardiol 1993;21:1638-44.). Univariate and multivariable Cox proportional hazards models were used to evaluate the associations between the outcome measures.
For building a first Cox regression model, we thereby used a stepwise procedure with an entry level of 0.05. The p value for staying in the model was set at 0.05. Event probabilities were estimated with the Kaplan-Meier method and the log-rank test was used for comparisons of curves. Additional statistical analyses were used for exploration, description, and interpretation. Comparisons between groups were done by the Students t-test, U-test or chisquare test as applicable. All p-values reported are two-sided, a p-value <0.05 was considered significant.

Receiver operator characteristic (ROC) curves were constructed to assess the sensitivity and specificity of biomarkers throughout the concentrations to detect respective endpoints. All statistical calculations were performed using the SPSS statistical software package (version 14.0; SPSS Inc., Chicago, IL).

### Results

Demographic characteristics of the overall study cohort are shown in table 1. During a maximum follow-up of up to 60 months (median 51 months) after enrolment, 27 patients had first malignant tachyarrhythmic events CT-proET1 levels were significantly higher in patients with (median: 90pmol/L) compared to patients without a first malignant tachyarrhythmic event (median: 67pmol/L; p=0.003). In contrast, no differences were found between groups for BNP (p=0.55) (Table 1).

### Predictors of first malignant tachyarrhythmic events

Malignant tachyarrhythmias occurred at a median of 18 months after study enrolment (IQR: 8-41 months). CT-pro-ET-1 levels, NYHA class, increased jugular venous pressure, and treatment with betablockers significantly contributed to univariate Cox regression models to predict the primary endpoint. Neither BNP, nor systolic blood pressure, LV enddiastolic diameter, LV-EF, or the presence of a left bundle branch block (LBBB) predicted risk of malignant tachyaarrhythmias requiring ICD therapy (Table 2). ICD patients have been stratified into two groups according to CT-proET-1 or BNP levels and Kaplan Meier analysis has been performed. Risk of tachyarrhythmia was significantly higher in patients with CT-proET-1 plasma levels >73pmol/L (median) than in patients with CT-proET-1 values below this cut point (logrank test, p<0.001, Fig. 1A). No differences were found in patients with BNP plasma levels ≤183 pg/mL (median) or >183pg/mL using malignant tachyarrhythmia as endpoint (logrank test, p=0.10, Fig. 1B). No significant associations of LV ejection fraction and CT-proET-1 levels were found (Fig. 2). The area under the ROC curve that used CT-proET-1 levels to predict the occurrence of a first malignant tachyarrhythmia was 0.69 (Fig. 1A, insert). The optimum concentration of CT-proET-1 for the calculation of positive and negative predictive accuracy as obtained from the ROC curve was 75.5pmol/L (sensitivity 80.8%, specificity 64.4%, negative predictive value 92.1%, positive predictive value 39.6%, and a positive likelihood ration of 2.27).
CT-proET-1 plasma levels significantly contributed to multivariable Cox regression models predicting the risk of first malignant tachyarrhythmia. Risk ratio has been calculated per pmol/L increment of CT-proET-1 levels (Table 2). Using log CT-proET-1 in statistical analysis did not change obtained results. Multivariable Cox regression analysis revealed that treatment with beta blockers indicates favourable prognosis (Table 2).

**Table 1**

| | | | | |
|---|---|---|---|---|
| Clinical characteristics of patients with implantable cardioverter/defibrillator implantation at study entry. | | | | |

| **Variable** | **All patients** | **Malignant tachyarrhythmia** | | |
|---|---|---|---|---|
| | | **Event** | **Event-free** | **P=** |
| Number | 123 | 27 | 96 | |
| Age, years | 63±12 | 64±13 | 63±12 | 0.66 |
| Male sex (%) | 104 (85) | 21 (78) | 83 (87) | 0.36 |
| Weight, kg | 82±14 | 82±13 | 82±14 | 0.83 |
| Body mass index, kg/m² | 26.9±4.2 | 27.6±4.0 | 26.6±4.2 | 0.29 |
| Systolic blood pressure, mmHg | 132±21 | 130±18 | 132±22 | 0.53 |
| **Dyspnea** NYHA class I/II (%) | 75(61) | 13 (48) | 62 (65) | 0.08 |
| NYHA class III (%) | 45 (37) | 12 (44) | 33 (34) | |
| NYHA class IV (%) | 3 (2) | 2 (7) | 1 (1) | |
| **Cause of HF** non-ischemic (%) | 63 (51) | 15 (56) | 48 (53) | 0.83 |
| **Clinical chemistry** | | | | |
| Hemoglobin, g/L | 141±16 | 144±17 | 140±16 | 0.21 |
| Sodium, mmol/L | 139±3 | 139±3 | 140±4 | 0.29 |
| Potassium, mmol/L | 4.0 ±0.4 | 4.0±0.5 | 4.0±0.4 | 0.61 |
| Magnesium, mmol/L | 0.8±0.1 | 0.8±0.1 | 0.8±0.1 | 0.54 |
| Creatinine clearance, mL/min | 90±37 | 89±37 | 90±38 | 0.89 |
| **CT pro-endothelin 1,** pmol/L (median) | 73 | 90 | 67 | 0.003 |
| IQR | 59-95 | 76-99 | 57-92 | |
| **B-type natriuretic peptide,** pg/mL (median) | 183 | 221 | 177 | 0.55 |
| IQR | 77 - 395 | 75-482 | 78-357 | |
| **12-lead electrocardiogram (%)** | | | | |
| Sinus rhythm | 69 (55) | 15 (56) | 54 (56) | 1.0 |
| Atrial fibrillation | 31 (25) | 7 (26) | 24 (25) | 1.0 |
| Pacemaker stimulation | 37 (30) | 6 (22) | 31 (32) | 0.35 |
| Left bundle branch block | 42 (34) | 9 (33) | 33 (34) | 1.0 |
| **Echocardiographic study** | | | | |
| LV EDD, mm | 66±10 | 67±9 | 65±11 | 0.45 |
| LV ejection fraction, % | 29±10 | 28±9 | 29±10 | 0.53 |
| **Medication** | | | | |
| Diuretics (%) | 103 (83.7) | 23 (85.2) | 80 (83.3) | 1.00 |
| ACE-I, ARB (%) | 100 (81.3) | 19 (70.4) | 81 (84.4) | 0.16 |
| Betablocker (%) | 92 (74.8) | 15 (55.6) | 77 (80.2) | 0.01 |
| Digitalis (%) | 76 (61.8) | 18 (66.7) | 58 (60.4) | 0.66 |
| Spironolactone (%) | 43 (35.0) | 7 (25.9) | 36 (37.5) | 0.36 |
| Amiodarone (%) | 29 (23.6) | 5 (18.5) | 24 (25.0) | 0.61 |
| Satins (%) | 46 (37.4) | 7 (25.9) | 39 (40.6) | 0.18 |
| Class I antiarrhythmics (%) | 4 (3.3) | 1 (3.7) | 3 (3.1) | 1.00 |

| | | | | |
|---|---|---|---|---|
| NYHA, New York Heart Association; LV, left ventricular; CT, C-terminal, EDD, enddiastolic diameter, ACE-I, angiotensin converting enzyme inhibitor, ARB, angiotensin receptor blocker. Statistical analysis has been performed as outlined in the statistics section. Values are displayed as mean±SD or as indicated. | | | | |

**Table 2: Tachyarrhythmic events in ICD patients during follow-up**

| | | **First malignant tachyarrhythmic event** | | |
|---|---|---|---|---|
| Parameter | | Hazard ratio Point estimate (95% CI) | P univariate | P multivariabl e |
| Age, years | | 1.01 (0.98-1.05) | 0.51 | - |
| Male sex | | 0.58 (0.23-1.43) | 0.24 | - |
| Body mass index, kg/m² | | 1.05 (0.95-1.16) | 0.32 | - |
| Medical history | Coronary artery disease/prior myocardial infarction | 0.83 (0.38-1.78) | 0.63 | - |
| | Successful resuscitation prior to ICD implantation | 0.52 (0.20-1.36) | 0.18 | - |
| | Syncope prior to ICD implantation | 1.54 (0.72-3.29) | 0.27 | - |
| | Presence of malignant arrhythmias before enrolment | 1.14 (0.51-2.55) | 0.74 | - |
| | ICD for primary prevention of malignant arrhythmias | 1.13 (0.52-2.43) | 0.76 | - |
| | Smoking | 0.63 (0.22-1.83) | 0.40 | - |
| | Hypertension | 0.57 (0.26-1.28) | 0.17 | - |
| | Diabetes mellitus | 1.45 (0.68-3.09) | 0.33 | - |
| | Increased jugular venous pressure | 3.92 (1.34-11.48) | 0.01 | 0.38 |
| | Systolic blood pressure, mmHg | 0.99 (0.97-1.01) | 0.50 | - |
| | New York Heart Association | 2.16 (1.13-4.14) | 0.02 | 0.08 |
| Medication | On treatment with ACl or ARBs | 0.48 (0.21-1.09) | 0.08 | - |
| | On treatment with loop diuretics | 1.48 (0.69-3.15) | 0.31 | - |
| | On treatment with spironolactone | 0.59 (0.25-1.40) | 0.23 | - |
| | On treatment with betablockers | 0.29 (0.14-0.63) | 0.002 | 0.01 |
| | On treatment with amiodarone | 0.81 (0.31-2.15) | 0.68 | - |
| | On treatment with statins | 0.53 (0.22-1.25) | 0.14 | - |
| 12 lead ECG | Presence of atrial fibrillation | 1.06 (0.45-2.50) | 0.90 | - |
| | Left bundle branch block | 0.93 (0.42-2.06) | 0.85 | - |
| Echocardiography | LV end-diastolic diameter, mm | 1.02 (0.98-1.05) | 0.32 | - |
| | LV ejection fraction, % | 0.99 (0.95-1.02) | 0.42 | - |
| Clinical chemistry | Haemoglobin, g/L | 1.02 (0.99-1.04) | 0.27 | - |
| | Potassium, mmol/L | 0.71 (0.30-1.68) | 0.43 | - |
| | Magnesium, mmol/L | 0.24 (0.002-27.08) | 0.55 | - |
| | Creatinine clearance (ml/min) | 0.99 (0.98-1.01) | 0.59 | - |
| | C-terminal pro-endothelin-1, pmol/L | 1.03 (1.01-1.04) | <0.001 | 0.02 |
| | B-type natriuretic peptide, pg/mL | 1.001 (1.000-1.002) | 0.19 | - |

| | | | | |
|---|---|---|---|---|
| Hazard ratios for the primary endpoint (first malignant tachyarrhythmic event) during 60 months of follow-up (n=123; LV, left ventricular; ICD, implantable cardioverter defibrillator) | | | | |

## Claims

1. An in vitro method for risk stratification for the occurrence of malignant tachyarrhythmias for a patient having a cardiac disease comprising:
• determining the level of Pro-Endothelin-1 (ProET-1) or fragments thereof of at least 12 amino acids in a sample obtained from a patient.

2. *An in vitro* method according to claim 1, wherein said level of Pro-Endothelin-1 (ProET-1) or fragments thereof of at least 12 amino acids is correlated to the risk of malignant tachyarrhythmias requiring implantation of an implantable cardioverter defibrillator (ICD), and/or an antiarrythmic hybrid therapy.

3. An *in vitro* method according to claim 2, for primary prevention of sudden arrythmic death.

4. A method according to claim 2 or 3, wherein said correlating step comprises comparing said level of Pro-Endothelin-1 (ProET-1) or fragments thereof of at least 12 amino acids to a threshold level, whereby, when said level of Pro-Endothelin-1 (ProET-1) or fragments thereof of at least 12 amino acids or fragments thereof exceeds said threshold level, said patient is at high risk of tachyarrhythmia

5. A method according to claim 4, wherein said threshold level is at about 73 +/-20% pmol/L, if CT-proET-1 is used as a Pro-Endothelin-1 (ProET-1) fragment.

6. A method according to claims 1-5, wherein the cardiac disease is heart failure of either ischemic or non-ischemic etiology.

7. A method according to claims 1-6, wherein the patients have a LV ejection fraction ≤ 45%.

8. A method according to any of the preceding claims, wherein said sample is selected from the group comprising a blood sample, a serum sample, and a plasma sample.

9. A method according to any of the preceding claims, further comprising combining said level of Pro-Endothelin-1 (ProET-1) or fragments thereof of at least 12 amino acids with the level/condition of one or more additional prognostic markers, parameters or factors, whereby the combination of said level of Pro-Endothelin-1 (ProET-1) or fragments thereof of at least 12 amino acids with said level/condition of additional prognostic marker(s), parameter(s) or factor(s) increases the predictive value for risk of said method.

10. A method according to claim 11, wherein the additional prognostic marker, parameter or factor is selected from a group comprising all parameters listed in Table 2, which exhibit a p<0.05 in the univariate or multivariate analysis, whereby BNP is only an example for proBNP or fragments thereof of at least 12 amino acids including BNP or NT-proBNP.

11. A method according to claims 1 to 10, wherein said level of Pro-Endothelin-1 (ProET-1) or fragments thereof of at least 12 amino acids is measured with a sandwich immuno assay.

12. A method according to claims 1-10, wherein the Pro-Endothelin-1 fragment is CT-proET-1.
